# EUROPEAN PATENT SPECIFICATION

(11) **EP 3 645 602 B1**
(45) Date of publication and mention of the grant of the patent: **07.06.2023**
(21) Application number: 18799745.7
(22) Date of filing: 06.11.2018
(51) Int. Cl.: C08G 63/676, C09J 167/06, C09J 171/02, A61F 13/02, C09J 133/06

(54) **ADHESION MATERIALS AND METHODS OF MANUFACTURE**
HAFTMATERIALIEN UND VERFAHREN ZUR HERSTELLUNG
MATÉRIAUX D'ADHÉRENCE ET PROCÉDÉS DE FABRICATION

(30) Priority: 06.11.2017 PT 2017073487
(43) Date of publication of application: 06.05.2020
(73) Proprietor: BestHealth4U, Lda, 4700-312 Braga (PT)
(72) Inventor: OLIVEIRA, Nélson Duarte Mendes, 4770-210 Joane (PT); FERREIRA, Sónia Carla Abrantes, 1750-375 Lisboa (PT)
(74) Representative: Harrison, Robert John
(86) International application number: PCT/EP2018/080359
(87) International publication number: WO 2019/086716

(56) References cited:
- EP-A1- 2 789 335
- EP-A1- 2 853 573
- US-A1- 2011 105 604

## Description

### Field of the Invention

The invention relates generally to adhesion materials and a method of their production. More particularly, the present invention relates to a new type of adhesive including the adhesion material that binds a pad, devices or other objects to the skin through hydrogen bonds.

### Background of the Invention

An adhesive is a product that can be applied to almost every type of medical device to bind the medical device to a skin of a person. For example, the adhesive can be applied to a simple wound dressing to enable the wound dressing to bind more tightly to the skin or to a more complex product, such as but not limited to, an ostomy bag that needs to be held in place on the skin.

In the market of medical adhesives there are several solutions known that comprise a mixture of one or more adhesion materials, such as Acrylate, hydrocolloid or silicone. In recent years, there have been extensive advances in the adhesives industry which have produced new advances in the adhesion materials in a widening spectrum. For example, it is possible to produce variations of the adhesion material by adding different materials to the final composition and thereby enable properties of the adhesion materials to be tailored to meet specific application requirements, including biostability, sterilization effects, and mechanical properties.

The main types of adhesion materials used currently in the medical device area are cyanoacrylate, fibrin glue, GRF (Gelatin, resorcinol, formaldehyde), hydrogel and hydrocolloid and other adhesion materials with small market share or in research as the silicones, poly ethylene glycol, albumin, chitosan, gelatin. These adhesion materials compositions have known disadvantages, such as stripping (removing the adhesion material from the surface), mechanical injury due to tension, allergic contact resulting in dermatitis, folliculitis and skin maceration.

Beside the afore-mentioned disadvantages, a hydrocolloid dressing also presents the further disadvantages that the hydrocolloid dressing may dislodge from contact with the skin due to shearing or friction. The hydrocolloid dressing is also not recommended for use with infected wounds due to fluid discharge as the hydrocolloid dressing will also dislodge from the wound with heavy drainage. Finally, the odor when the hydrocolloid dressing is removed can be very strong and the removal may injure fragile skin.

### Prior Art

As was explained before, in this market it is possible to find several types of adhesion materials that use different strategies to stick to the skin, such as chemically, electrostatically or mechanically. The chemical adhesion materials comprise a large quantity of materials, such as but not limited to acrylic, styrene-isoprene-styrene block copolymers, high molecular weight polyisobutylenes loaded with glycols, fat, oil, glycerol esters or surfactants.

Silicone adhesives are also widely employed as skin friendly adhesives. In the patent document, EP 2853573 A1, for example, a diol was reacted with dicarboxylic acid through the polycondensation method, to develop an adhesion material which is friendly towards the skin.

According to the US patent number US 5308887 A and international patent application No WO 2017/021448 A1 there are some pressure sensitive adhesives that are applied in strips, swirls, or waves. This type of adhesive refers to any releasable adhesive or releasable tenacious means. Suitable adhesion materials include, water-based pressure-sensitive adhesion materials, such as acrylate adhesives, natural rubbers, synthetic rubbers, vinyl acetates, silicones and polyurethanes. These adhesion materials can be based on emulsion or solvent-borne adhesives of natural or synthetic polyisoprene, styrene-butadiene, or polyacrylate, vinyl acetate copolymer.

Another type of adhesion material called hot melt adhesives are also known. According to the patent number EP 2756830 B1, a pressure-sensitive hot melt adhesive comprises substantially saturated block copolymer, tackifying resin, and polybutene. This hot melt composition exhibits adhesive stability when exposed to ultra-violet light and is stable when contacted with plasticized surfaces. These block copolymers include linear or radial co-polymer structures having the formula (A-B) wherein block A is a polyvinylarene block and block B is a poly(monoalkenyl) block. Block A, includes polystyrene, polyalpha-methylstyrene and polyvinyltoluene. Block B, can include, conjugated diene elastomers such polybutadiene or polyisoprene or hydrogenated elastomers such as ethylene butylene or ethylene propylene or polyisobutylene.

Commercial examples of these types of block copolymers include Kraton^{™} elastomers from Shell Chemical Company, Vector ^{™} elastomers from Dexco, Solprene^{™} from Enichem Elastomers and Stereon ^{™} from Firestone Tire & Rubber Co.; hot melt adhesive based on olefin polymers and copolymers where in the olefin polymer is a terpolymer of ethylene and co-monomers, such as vinyl acetate, acrylic acid, methacrylic acid, ethyl acrylate, methyl acrylate, n-butyl acrylate vinyl silane or maleic anhydride. Commercial examples of these types of polymers include Ateva (polymers from AT plastics), Nucrel (polymers from DuPont), Escor (from Exxon Chemical).

Another type of adhesion material existing in the market is called a gecko solution. This gecko solution is a combination of microfibers and nanofibers that connect to the skin using a mechanical and electrostatic connection that results from the cumulative effect of van der Waals forces between the millions of setae on the feet pads in intimate contact with the surface the gecko is climbing on. The patent application US No 2014/0272272A1 uses an electrostatic dry adhesive device having a micro-structured element formed directly into a contact surface of an electrostatic adhesive. The micro-structure in the form of microwedges can be molded into surface of an electrostatic adhesive. This document refers the use of at least one electrode that comprises a conductive mesh or fabric.

US Patent Application Publication No 20120288680 A1 uses a micro-featured and nano-featured surface, and a compliant surface having a hardness of about 60 Shore A or less. This type of adhesion composition needs flat surfaces for adhesion, such as polymer, metal or other.

The study "Adhesion and friction of an isolated gecko setal array: The effects of substrates and relative humidity", by Y. Tian, showed that water affects the adhesion of the setal array by changing the Hamaker constant, and when the gecko encounters a surface with a higher surface energy or a more humid ambient environment, the gecko enjoys climbing more flexibly. Unfortunately, and according to the study "Surface free energy of the human skin and its critical surface tension of wetting in the skin/surfactant aqueous solution/air system", by Krawczyk J, the skin surface is a low-energetic one. The critical surface tension of the skin wetting depends on the type of surfactant. In this respect, non-ionic surfactants seem to be the most appropriate.

Maleic anhydride has also been studied as an adhesion material. The researcher Hernández Sierra JF studied the application of this type of materials in *"Bactericidal capacity of silver nanoparticles associated with Gantrez S-97 on Streptococcus mutans".* He concluded that the addition of Gantrez 2% to silver nanoparticles does not alter its antimicrobial effect. [Hernández, Sierra JF, Salas, Lopez EK, et al. "Bactericidal capacity of silver nanoparticles associated with Gantrez S-97 on Streptococcus mutans."; 2010 Winter;35(2):183-5.] Anastasia Ripolin studied the application of this type of material in microneedle patches to be used in humans. She concluded that the use of such larger patches by patients can be successful, potentially opening up the possibility for a significant expansion of the size of the market for transdermal drug delivery. [Ripolin, Anastasia; James, Quinn; et al. "Successful application of large microneedle patches by human volunteers ". International Journal of Pharmaceutics, Vol.521, Issues 1-2, 15 April 2017, 92-101.] E. Blanco Garcia has studied microspheres (Ms) based on zein (ZN) and Gantrez^{®}AN119 (PVMMA) that were prepared by spray-drying and coated with a pH-sensitive polymer (Eudragit^{®} FS30D) in intestinal inflammatory disorders. The authors concluded that ZN/PVMMA microspheres is a serious alternative for delivering the medicine to reduce the inflammatory activity at intestinal regions affected by inflammatory bowel diseases. [Garcia, E. Blanco; Espinar, F.J. Otero; "Development and characterization of antiinflammatory activity of curcumin-loaded biodegradable microspheres with potential use in intestinal inflammatory disorders". International Journal of Pharmaceutics, Volume 518, Issues 1-2, 25 February 2017, 86-104]. This affinity has been studied and similar solutions patented by other authors, one of the studies showed the capability of the maleic anhydride to connect with the hydroxy or hydroxyl group.

The functionalization of polymers with this additive material is disclosed in the U.S. Patent No. 6,451,919 B1 (1998). The patent teaches how the polyolefins can be functionalized with maleic anhydride and at least one high-boiling ester of the latter, and a process for their preparation in high yields, with high degrees of functionalization, without the production of by-products of cross-linking or degradation is disclosed.

In US. 8,940,132 are disclosed the interface modifiers, e.g., surface active agents, that may be used in functionalized polymers, preferably maleic anhydride grafted polyolefins, to improve the adhesion with cellulosic pulp fibers.

US 8,940,278 focused in the efficacy enhancing agent that preferred copolymers of maleic anhydride or acid with another polymerizable ethylenically unsaturated monomer. The maleic anhydride was also used in the US 8,906,406 and 8,907,153 for a mucoadhesive medicinal containing at least one active substance, in which a matrix comprises a mixture that comprises sodium carboxymethyl cellulose, polyvinyl alcohol and hydroxypropylmethyl cellulose. The adhesion material comprises at least one type of cellulose, derivatives, and for example maleic acid anhydride. As matrix-forming polymers which can be components of a mucoadhesive formulation, the following polymers were taken into consideration as raw materials: Polyvinyl alcohol (e.g. Mowiol^{(R)}; cellulose derivatives, especially the types of Polyox 10, Polyox 80, Polyox 205, Polyox 301, Polyox 750 (made by the firm of Union Carbide); copolymers of methyl vinyl ether and maleic acid anhydride (Gantrez-Copolymers, especially the types of ES, MS, S; by the firm of ISP Global Technologies GmbH). According to the US. Pat. No. 8,940,132, it is preferably, the surface-active agent(s) presented in an amount greater than 2% by weight and less than 15% by weight of the entire composition of the composite, and more preferably in an amount less than or equal to 10% by weight.

Further patent applications are known which describes similar compositions. For example International Patent Application No. WO2017/075320 describes an antimicrobial adhesive made of silicone.

International patent application No. WO2010043346 teaches an aqueous solution of materials, or materials solubilized in conventional cosmetically or dermopharmaceutically acceptable solvents selected from the group consisting of ethanol, propanol, isopropanol, propylene glycol, glycerin, butylene glycol, or polyethylene glycol and mixtures thereof.

US patent application No. US2013/0052236A1 teaches an adhesive based on mucoadhesive aspect comprises an alginate, phloroglucinol, maleic acid and an alkyl vinyl ether, as well as glycerol.

US patent application No. US2007/0009582A1 teaches a plasticizer. This is only used to reduce the melting temperature. The adhesive is made only from hydrophobic block(s) consists essentially of polymerized (meth)acrylic ester. The glycerol here is used as said aqueous solution and not to be used to create hydrogen bonds.

European Patent Application No 2 853 573 (Nitto Denko Corp) teaches the preparation of skin-friendly adhesives and patches. In particular, this patent application discloses a method of preparing an adhesive material by mixing polyethylene glycol with a dimer diol and dimer acid at a temperature of 180°C under vacuum for two hours. The adhesive materials disclosed in this application do not include citric acid, ascorbic acid, a sugar alcohol or sugar, glycerol and lauric acid or oleic acid.

US Patent Application Publication No. US 2011/0105604 (Sondgeroth et al) teaches a cleansing and moisturizing composition that uses strong cationic surfactants and strong anionic surfactants to deposit moisturizers onto a user's skin. This publication fails, however, to teach the use of the composition as an adhesive.

### Summary of the Invention

The adhesion material presented in this document is used in a novel type of adhesion material to secure a medical device to a skin of a human or an animal. This adhesion material was developed to overcome the main disadvantages of the current types of adhesion materials, namely the mechanical injury due to tension, allergic contact dermatitis, folliculitis and maceration. To overcome the disadvantages of the current adhesion materials, the adhesion material developed has the capability to bind to the water molecules that exist on top of the skin of the human or animal and not directly to the skin itself. To achieve this, the adhesion material uses hydrogen bonds. The binding with the hydrogen bonds reduces the number of skin cells that are removed on removal of the material as known in the prior art adhesion material, since the adhesion material taught in this document is not in direct contact with the skin.

As was explained above, the adhesion material developed is based on the principal of hydrogen bonds. The hydrogen bonds are an attractive force between the hydrogen atom attached to an electronegative atom of one molecule and an electronegative atom of a different molecule. Usually the electronegative atom is oxygen, nitrogen, or fluorine, which has a partial negative charge. The hydrogen has the partial positive charge. This bond is not nearly as strong as normal covalent bonds within a molecule (about 1/10 as strong). However, this strength is enough to have important ramifications on the properties of water.

To achieve a good adhesion between the adhesion material of this document and the skin, the atoms of O···H need to be at distance of around 2.06 Å apart. At this distance, there exists both an electrostatic and covalent component of the bonding. If the distance between the two atoms increases or the amount of water is reduced, the adhesion strength of the adhesion material will be reduced to substantially zero, which will help to remove the medical device that is held to the skin by the adhesion material of this document. With this, the problems known in the art such as stripping, mechanical injury due to tension, allergic contact dermatitis, folliculitis, maceration are eliminated or at least substantially reduced.

In one aspect, the adhesion material is a composition that comprise a mixture or the reaction of polyethylene glycol (PEG), preferably from 200 to 8000 g/mol, with a sugar, such as sucrose, maltose, lactose or fructose, and/or a sugar alcohol (also called polyhedric alcohol) such as xylitol (152 g/mol) or sorbitol, glycerol (92 g/mol), castor oil (150 g/mol), ascorbic acid (176 g/mol), citric acid (192 g/mol), lignin (1513 g/mol), oleic acid (282 ,47 g/mol) and lauric acid (200 g/mol). It is known that PEG is a linear molecule in which the OH groups are localized at the end of the molecular chain. This localization will allow controlling the flexibility of the final material. The other materials due to their quantity of the hydroxy groups -OH will allow an increase the hydrogen bonds in the adhesion material and thus between the adhesion material and the water in the skin. The lauric acid or oleic acid will provide water repellence.

To the previous composition is added one of the following compounds in manner to promote the necessary adhesion: Polypropylene grafted with maleic anhydride or Polyethylene grafted with maleic anhydride or poly (ethylene-alt-maleic anhydride) or polystyrene-block-poly(ethylene-ran-butylene)-block-polystyrene-graft-maleic anhydride or polyisoprene-graft-maleic anhydride or poly(methyl vinyl ether-alt-maleic anhydride) or poly(isobutylene-alt-maleic anhydride) or poly(maleic anhydride-alt-1-octadecene) solution.

The materials presented above have on their structure a molecule that has affinity with water. This affinity is given mainly due to maleic anhydride molecule. This molecule is reported by Arieny Rodrigues, in the study "Effect of compatibilization and reprocessing on the isothermal crystallization kinetics of polypropylene/wood flour composites", in Polimeros, vol.23, no.3, São Carlos, 2013. The presence of a compatibilizer between two materials, such as a polymer and wood, decreases the interfacial tension, increasing the adhesion between the two materials and provides wettability, thus preventing agglomeration. In the market, there exist several materials grafted with maleic anhydride. It is possible to find materials with a concentration of this additive of between 1 to 10 % in weight.

There are a large number of materials that are grafted with these additive and suitable ones of the materials can be selected by defining requirements according with the end application. To be more specific the material will be selected based on the level of erythema (redness of skin) caused by the material, the capability to create the adhesion on the skin at the temperature of the body (i.e. between 37°C and 40°C) and the capability to support a weight of 1 kg during 3 days without losing adhesiveness.

Another factor that will be considered to select the material is the manufacturing technique. According to European Chemicals Agency (ECHA, https://echa.europa.eu/substance-information/-/substanceinfo/100.003.247), maleic anhydride may cause irritation to the respiratory tract, eyes, exposed and skin. Maleic anhydride is also a skin and respiratory sensitizer.

Lignin is another material that can be used in the adhesion material, since lignin is an amorphous natural polymeric material that is based on a phenylpropane derivate, with large number of hydroxyl groups on their molecule chain, one of the most abundant materials and renewable resources on earth. The lignin-based adhesives have potentials for engineering applications due to their environmental suitability and economic and technical feasibility.

The manufacturing techniques that can be used to add the adhesion material to the medical device are the nano-spray, nano-printing and polymer brush. The polymer brush is a technique that described by William J. Brittain in the work "A Structural Definition of Polymer Brushes", Journal of Polymer Science: Part A: Polymer Chemistry, Vol. 45, 3505-3512 (2007). A polymer brush is an array of macromolecular chains which is attached or tethered to a surface and in sufficient proximity so that the unperturbed solution dimensions (in a good solvent) of the chains are altered. In the study, Brittain inferred that, to obtain a good alignment, it is necessary that the polymers have a low molecular weight of 14,500 g/mol.

A non-limiting example of the adhesive patch with the adhesion material will now be described. The adhesive patch has the following layers:
a) an external layer made of a water proof and breathable material (as for example ePTFe, silicone, thermoplastic polyurethane (TPU), polyether block amide (PEBA) or ethylene-vinyl acetate (EVA), with a thickness between 50 µm -500 µm);
b) an intermediate layer made of non-woven or woven fabric (non-limiting examples include polyamide, polyester, cotton, or fibers of polypropylene or polyethylene);
c) an inner layer of the adhesion material.

The inner layer is made of the adhesion material taught above and can interact with the water that exists in the skin. This adhesion material is composed by a mixture or a reaction result of polyethylene glycol and citric acid and ascorbic acid and glycerol as well as lauric acid and/or oleic acid with polypropylene grafted with maleic anhydride or Polyethylene grafted with maleic anhydride or) or poly (ethylene-alt-maleic anhydride) or polystyrene-block-poly(ethylene-ran-butylene)-block-polystyrene-graft-maleic anhydride or polyisoprene-graft-maleic anhydride or poly(methyl vinyl ether-alt-maleic anhydride) or poly(isobutylene-alt-maleic anhydride) or poly(maleic anhydride-alt-1-octadecene) or poly(ethylene glycol)methyl ether methacrylate solution polyethylene glycol, with a polyol.

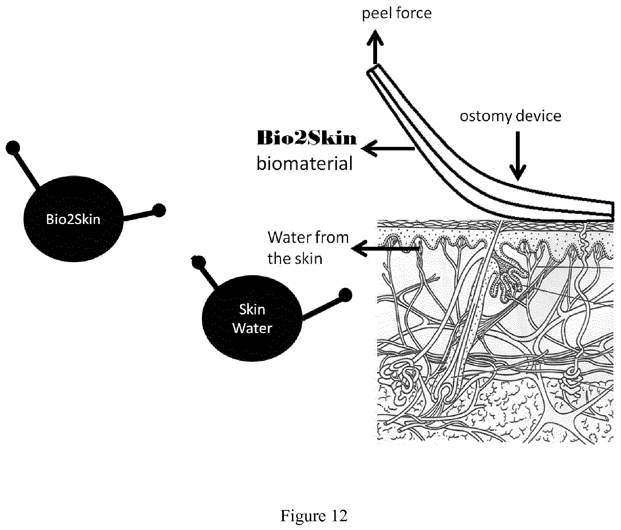

The process used to react different polyols is esterification. The materials polyethylene glycol, citric acid, ascorbic acid, glycerol, Xylitol as well as lauric acid and/or oleic acid are added together or separated, mixed at temperatures between 80°C to 200°C, for 1h to 24h, using pressures of between a near vacuum (zero atmosphere) and 202,650 Pa (2 atmospheres). As an example, the structure of the polyol could be like the one presented in figure 12.

In one aspect, the adhesion material comprises a mixture of polyethylene glycol and citric acid and ascorbic acid and glycerol and xylitol and lauric acid and/or oleic acid.

In another aspect, the adhesion material comprises a mixture of polyethylene glycol and citric acid and ascorbic acid and glycerol and xylitol and lauric acid/ oleic acid with polypropylene grafted with maleic anhydride.

In another aspect the adhesion material comprises, a mixture of polyethylene glycol and citric acid and ascorbic acid and glycerol and xylitol and lauric acid/oleic acid and the material resulted by their reaction of the different polyols with Polyethylene grafted with maleic anhydride.

In an aspect the adhesion material comprises a mixture of polyethylene glycol and citric acid and ascorbic acid and glycerol and xylitol and lauric acid/oleic acid with poly (ethylene-alt-maleic anhydride).

In an aspect, the adhesion material comprises, a mixture of polyethylene glycol and citric acid and ascorbic acid and glycerol and xylitol and lauric acid/oleic acid with polystyrene-block-poly(ethylene-ran-butylene)-block-polystyrene-graft-maleic anhydride or polyisoprene-graft-maleic anhydride or poly (methyl vinyl ether-alt-maleic anhydride).

In an aspect, the adhesion material comprises a mixture of polyethylene glycol and citric acid and ascorbic acid and glycerol and xylitol and lauric acid/oleic acid with poly(isobutylene-alt-maleic anhydride).

In an aspect, the adhesion material comprises a mixture of polyethylene glycol and citric acid and ascorbic acid and glycerol and xylitol and lauric acid/oleic acid with poly (maleic anhydride-alt-1-octadecene).

In an aspect, the adhesion material comprises a mixture of polyethylene glycol and citric acid and ascorbic acid and glycerol and xylitol and lauric acid/oleic acid with poly(ethylene glycol)methyl ether methacrylate solution.

In an aspect, the adhesion material comprises a mixture of 14% to 16% in weight of polyethylene glycol and 18% to 21% in weight of citric acid and 2% to 4% in weight of ascorbic acid and 4% to 6% in weight of glycerol and 5% to 7% in weight of xylitol and 1% to 2% in weight of oleic acid.

In one aspect, the adhesion material comprises a mixture of 14% to 16% in weight of polyethylene glycol and 18% to 21% in weight of citric acid and 2% to 4% in weight of ascorbic acid and 4% to 6% in weight of glycerol and 5% to 7% xylitol and 1% to 2% in weight of lauric acid.

In a further aspect, xylitol can be replaced by another sugar alcohol such as sorbitol, as well as by a sugar such as fructose, lactose, maltose or sucrose.

In one aspect, the polyol resulted from the reaction of the different polyols can also be used, in this case is used between 48% and 52% in weight. The adhesion material has 48 % to 52% in weight of butyl ester of methyl vinyl ether grafted with maleic anhydride or Polypropylene grafted with maleic anhydride or Polyethylene grafted with maleic anhydride or poly (ethylene-alt-maleic anhydride) or polystyrene-block-poly(ethylene-ran-butylene)-block-polystyrene-graft-maleic anhydride or polyisoprene-graft-maleic anhydride or poly(methyl vinyl ether-alt-maleic anhydride) or poly(isobutylene-alt-maleic anhydride) or poly(maleic anhydride-alt-1-octadecene) or poly(ethylene glycol)methyl ether methacrylate solution.

### Brief description of the drawings

For easier understanding, the figures and tables representing preferential embodiments not intend to limit the object of the present description.

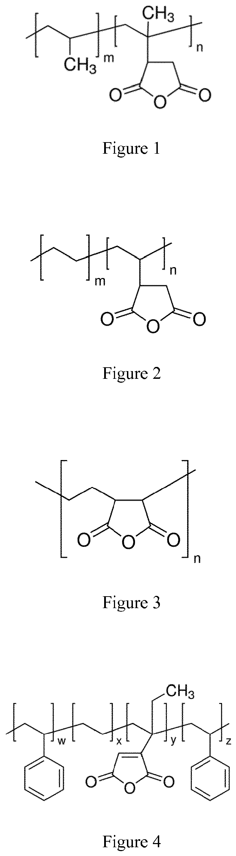

Fig. 1 shows the molecular structure of polypropylene grafted with maleic anhydride;
Fig. 2 shows the molecular structure of polyethylene grafted with maleic anhydride.
Fig. 3 shows the molecular structure of poly (ethylene-alt-maleic anhydride).
Fig. 4 shows the molecular structure of polystyrene-block-poly(ethylene-ran-butylene)-block-polystyrene-graft-maleic anhydride.

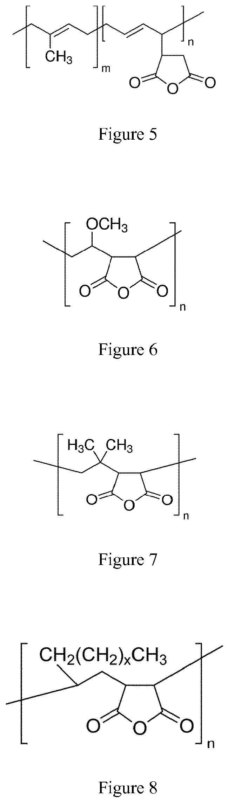

Fig. 5 shows the molecular structure of polyisoprene-graft-maleic anhydride.
Fig. 6 shows the molecular structure of poly(methyl vinyl ether-alt-maleic anhydride).
Fig. 7 shows the molecular structure of poly(isobutylene-alt-maleic anhydride).
Fig. 8 shows the molecular structure of poly(maleic anhydride-alt-1-octadecene).

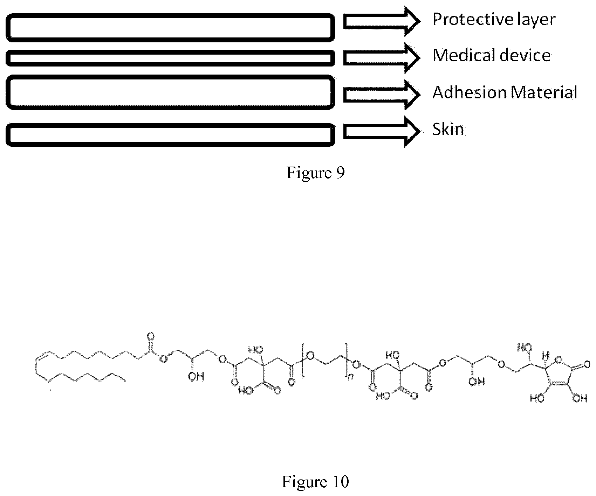

In Fig. 9 is an example of how the materials can be placed.
Fig. 10 shows the molecular structure resulted by the reaction between the different polyols.

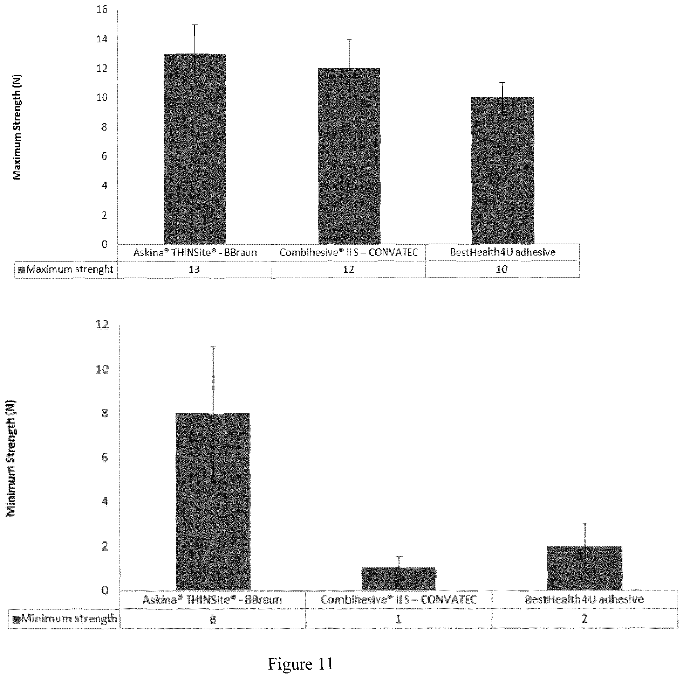

Fig. 11 show the results of adhesion strength of the adhesive comparing with products already in the market.
Fig. 12 show the material attached to the skin

### Detailed description of the Invention

### Example 1

In this example the application of the composition will be illustrated. The composition used in the adhesion material on the adhesive patch as shown in the figure 12, is a mixture of
- 14% to 16% by weight of polyethylene glycol 400;
- 18% to 21% by weight of citric acid;
- 2% to 4% by weight of ascorbic acid;
- 4% to 6% in weight of glycerol;
- 5% to 7% In weight of xylitol
- 1% to 2% in weight of oleic acid.

The biopolymer resulted from the reaction of the different polyols is mixed, in this case is used between 44% and 56% in weight with the maleic anhydride polymer. The adhesion material comprises 44% to 56% in weight of poly(methyl vinyl ether-alt-maleic anhydride). The materials to be use in the adhesion material are mixed by hand or with the help of a mixing machine at temperatures between 23°C and 90°C, and with a velocity of mixing between 1 rpm and 1000 rpm. Poly(methyl vinyl ether-alt-maleic anhydride) was selected because this material presents the molecular length necessary to interact with the deep zone of the skin.

A non-woven or a woven fabric, such as but not limited to polyamide, polyester, cotton, fibers of polypropylene or polyethylene, is used as the substrate for the adhesive patch. This substrate is a layer of a material with the capability to protect the adhesion material and the skin from atmosphere.

The adhesive patch was obtained as follows. Firstly, a protective layer with a thickness between 50 µm and 500 µm of silicone was applied at room temperature to one of the faces of the non-woven or woven fabric. This silicone layer is applied at room temperature using the technique of knife-coating and/or spraying and/or printing. The protective layer was cured up to a maximum of three hours at temperatures between 23°C to 100°C.

After this cure period the adhesion material was added to the other one of the faces of the non-woven or woven fabric by knife-coating, nano-printing or nano-spray.

### Example 2

This example illustrates a way of producing an adhesion material to be used in a hot and cold therapy adhesive patch. An adhesion material of a butyl ester of methyl vinyl ether grafted with maleic anhydride was selected because this composition presents the molecular length necessary to interact with the deep zone of the skin and has in its structure the maleic anhydride necessary to adhere to the skin.

The adhesion material can be made through the following process:
The composition to be used in the adhesion material is a mixture of:
   - 14% to 16% by weight of polyethylene glycol 400;
   - 18% to 21% by weight of citric acid;
   - 2% to 4% by weight of ascorbic acid;
   - 4% to 6% in weight of glycerol;
   - 5% to 7% In weight of xylitol
1% to 2% in weight of oleic acid. The polyol resulted from the reaction of the different polyols can also be used, in this case is used between 44% and 56% in weight. The adhesion material must have 44 % to 56% in weight of a butyl ester of methyl vinyl ether grafted with maleic anhydride.

The materials were mixed for between 1 min to 24 hours at a temperature between 23°C and 110°C, more preferably between 30-90°C. The materials were mixed between 1 min to 24 hours.

The adhesion material is added to the face of the adhesive patch that will be in contact with the skin through knife-coating, nano-printing or nano-spray.

A protective layer of polyether block amide (PEBA) with a thickness of between 50 µm and 500 µm is added to the external face of the medical device. The protective layer will be used to protect the adhesion material from the atmosphere. The protective layer will be added to the adhesive patch by painting or thermoforming.

### Example 3

This example is a way of applying the adhesive to a wound dressing that uses a non-woven or woven fabric. A protective material is also used to protect the adhesion material from the atmosphere, and the adhesion material will enable adhesion to the skin. Poly (ethylene-alt-maleic anhydride) was used as the adhesion material because this adhesion material has the capability to interact with the water in the skin, as described above.

This adhesion material was made through the following process:
The adhesion material to be used in the adhesive is a mixture of
- 14% to 16% by weight of polyethylene glycol 400;
- 18% to 21% by weight of citric acid;
- 2% to 4% by weight of ascorbic acid;
- 4% to 6% in weight of glycerol;
- 5% to 7% In weight of xylitol
- 1% to 2% in weight of oleic acid..

The polyol resulted from the reaction of the different polyols can also be used, in this case is used between 44% and 56% in weight. The adhesion material must have 44% to 56% in weight of poly(ethylene glycol)methyl ether methacrylate solution.

To mix the different components of the adhesion material, temperatures between 23°C and 110°C, and more preferably between 30-90°C, were used. The materials were mixed between 1 min to 24 hours. These components were mixed by hand or with a help of a mixer with a mixing velocity between 1 rpm and 1000 rpm for between 1 min to 2 hours.

The adhesion material was applied to one face of the non-woven fabric made of a polyamide or a layer of thermoplastic polyurethane (TPU) with thickness of between 50 µm and500 µm.

### Testing

Tests regarding the adhesion capability of the adhesives were made, and these are shown in Fig. 12. These tests were made according with the standard ASTM D 3359-97 "tape test". They compared the adhesion strength of the examples of this document invention with prior art materials, namely Combihesive IIS from Convatec and Askina "Thinsite" from BBraun. These tests evaluated the maximum and the minimum force needed to remove the adhesive. The figure shows that to remove the adhesive was necessary less force (10N) compared with the ones in the state-of-the-art (13N for Askina and 12 for Combihesive). Relatively to the minimum force the adhesive developed needed more force (2N) comparatively to the Convatec (1N) but need less force when compared to the product of BBraun (8N).

## Claims

1. An adhesion material for binding an object to a skin of a human or an animal, the adhesion material comprising a mixture of polyethylene glycol, citric acid, ascorbic acid, glycerol, at least one of a sugar or a sugar alcohol, as well as one of lauric acid or oleic acid.

2. An adhesion material according to the above claim, further comprising lignin.

3. An adhesion material according to claim 1 or 2, further comprising polypropylene grafted with maleic anhydride or polyethylene grafted with maleic anhydride or poly (ethylene-alt-maleic anhydride) or polystyrene-block-poly(ethylene-ran-butylene)-block-polystyrene-graft-maleic anhydride or polyisoprene-graft-maleic anhydride or poly(methyl vinyl ether-alt-maleic anhydride) or poly(isobutylene-alt-maleic anhydride) or poly(maleic anhydride-alt-1-octadecene) or polyethylene glycol)methyl ether methacrylate solution.

4. An adhesion material according to any one of the previous claims, wherein 44% to 56% in weight is the mixture of polyethylene glycol, citric acid, ascorbic acid, glycerol xylitol and lauric acid/oleic acid or the material resulted from the reaction between them and 44% to 56% of weight is polypropylene grafted with maleic anhydride or polyethylene grafted with maleic anhydride or poly (ethylene-alt-maleic anhydride) or polystyrene-block-poly(ethylene-ran-butylene)-block-polystyrene-graft-maleic anhydride or polyisoprene-graft-maleic anhydride or poly(methyl vinyl ether-alt-maleic anhydride) or poly(isobutylene-alt-maleic anhydride) or poly(maleic anhydride-alt-1-octadecene) or polyethylene glycol)methyl ether methacrylate solution.

5. The adhesion material according to any one of the previous claims further comprising:
• 14% to 16% by weight of polyethylene glycol 400;
• 18% to 21% by weight of citric acid;
• 2% to 4% by weight of ascorbic acid;
• 4% to 6% in weight of glycerol;
• 5% to 7% In weight of xylitol
• 1% to 2% in weight of oleic acid.

6. The adhesion material according to any one of the previous claims, wherein the molecular weight of polyethylene glycol is between 200 to 8000 g/mol,

7. The adhesion material according to any one of the previous claims, wherein the molecular weight of citric acid is 192 g/mol.

8. The adhesion material according to any one of the previous claims, wherein the molecular weight of ascorbic acid is 176g/mol.

9. The adhesion material according to any one of the previous claims, wherein the molecular weight of glycerol is 92 g/mol.

10. The adhesion material according any one of the previous claims, wherein the sugar alcohol is selected from the group of alcohols consisting of xylitol or sorbitol and the sugar is from the group of carbohydrates consisting of sucrose, lactose, maltose or fructose.

11. The adhesion material according to any one of the previous claims, where in the molecular weight of lauric acid/oleic acid is between 200 and 300 g/mol.

12. The adhesion material according to claim 3, further comprises 8 % to 12% in weight of, polypropylene grafted with maleic anhydride or polyethylene grafted with maleic anhydride or poly (ethylene-alt-maleic anhydride) or polystyrene-block-poly(ethylene-ran-butylene)-block-polystyrene-graft-maleic anhydride or polyisoprene-graft-maleic anhydride or poly(methyl vinyl ether-alt-maleic anhydride) or poly(isobutylene-alt-maleic anhydride) or poly(maleic anhydride-alt-1-octadecene) or polyethylene glycol)methyl ether methacrylate solution.

13. Use of the adhesion material of any one of claims 1 to 12 in a medical device, such as an adhesive patch, kinesiology bands or other product that need to be in contact with the skin.

14. Method for production of the adhesive material described in claims 1 to 12 wherein the polyethylene glycol, citric acid, ascorbic acid, sugar alcohol or sugar, glycerol and lauric acid/oleic acid are mixed at temperatures between 80°C to 200°C, during 2h to 24h, using pressures between zero and 2 atmospheres and with a mixing velocity between 1 to 2000 rpm.

15. Method according to claim 14 wherein the velocity of the mixing between polyethylene glycol, citric acid, ascorbic acid, glycerol and lauric acid/oleic aci d with polypropylene grafted with maleic anhydride or Polyethylene grafted with maleic anhydride or poly (ethylene-alt-maleic anhydride) or polystyrene-block-poly(ethylene-ran-butylene)-block-polystyrene-graft-maleic anhydride or polyisoprene-graft-maleic anhydride or poly(methyl vinyl ether-alt-maleic anhydride) or poly(isobutylene-alt-maleic anhydride) or poly(maleic anhydride-alt-1-octadecene) or poly(ethylene glycol)methyl ether methacrylate solution ranges between 1 rpm to 1000 rpm, with a temperature of mixture between 23°C and 100°C, during 1 minute to 24 hours.

16. An adhesive patch comprising an external layer made of a waterproof and breathable material, an intermediate layer made of a fabric, and an inner layer made of the adhesion material of any one of claims 1 to 12.

17. The adhesive patch of claim 16, wherein the external layer is at least one of ePTFe, silicone, TPU, PEBA, or EVA and/or the intermediate layer is made of one of non-woven or woven fabric, such as cotton.

## Patentansprüche

1. Adhäsionsmaterial zum Binden eines Objekts an die Haut eines Menschen oder eines Tieres, das Klebematerial umfasst ein Gemisch aus Polyethylenglykol, Zitronensäure, Ascorbinsäure, Glycerin, mindestens einem von einem Zucker oder einem Zuckeralkohol, sowie einer von Laurinsäure oder Ölsäure.

2. Adhäsionsmaterial nach dem vorstehenden Anspruch, ferner umfassend Lignin.

3. Adhäsionsmaterial nach Anspruch 1 oder 2, ferner umfassend mit Maleinsäureanhydrid gepfropftes Polypropylen oder mit Maleinsäureanhydrid gepfropftes Polyethylen oder Poly(ethylen-alt-maleinsäureanhydrid) oder Polystyrol-Block-Poly(ethylen-ran-butylen)-Block-Polystyrol-Pfropf-Maleinsäureanhydrid oder Polyisopren-Pfropfmaleinsäureanhydrid oder Poly(methylvinylether-alt-maleinsäureanhydrid) oder Poly(isobutylen-alt-maleinsäureanhydrid) oder Poly(maleinsäureanhydrid-alt-1-octadecen) oder Poly(ethylenglykol)methylethermethacrylat-Lösung.

4. Adhäsionsmaterial nach einem der vorhergehenden Ansprüche, bei welchem 44% bis 56% des Gewichts die Mischung aus Polyethylenglykol, Zitronensäure, Ascorbinsäure, Zitronensäure, Ascorbinsäure, Laurinsäure/Ölsäure oder das aus der Reaktion zwischen ihnen resultierende Material ist und 44 bis 56 Gew.-% mit Maleinsäureanhydrid gepfropftes Polypropylen oder mit Maleinsäureanhydrid gepfropftes Polyethylen oder Poly(ethylen-alt-maleinsäureanhydrid) oder Polystyrol-Block-Poly(ethylen-ran-Butylen)-Block-Polystyrol-Pfropf-Maleinsäureanhydrid oder Polyisopren-Pfropf-Maleinsäureanhydrid oder Poly(methylvinylether-alt-maleinsäureanhydrid) oder Poly(isobutylen-alt-maleinsäureanhydrid) oder Poly(maleinsäureanhydrid-alt-1-octadecen) oder Poly(ethylenglykol)methylether-Methacrylatlösung.

5. Adhäsionsmaterial nach einem der vorhergehenden Ansprüche ferner umfassend:
• 14 bis 16 Gew.-% Polyethylenglykol 400;
• 18 bis 21 Gew.-% Zitronensäure,
• 2 bis 4 Gew.-% Ascorbinsäure,
• 4 bis 6 Gew.-% Glycerin,
• 5 bis 7 Gewichtsprozent Xylitol
• 1 bis 2 Gew.-% Ölsäure.

6. Adhäsionsmaterial nach einem der vorhergehenden Ansprüche, bei welchem das Molekulargewicht des Polyethylenglykols zwischen 200 und 8000 g/mol liegt.

7. Adhäsionsmaterial nach einem der vorhergehenden Ansprüche, bei welchem das Molekulargewicht der Zitronensäure 192 g/mol beträgt.

8. Adhäsionsmaterial nach einem der vorhergehenden Ansprüche, bei welchem das Molekulargewicht der Ascorbinsäure 176g/mol beträgt.

9. Adhäsionsmaterial nach einem der vorhergehenden Ansprüche, bei welchem das Molekulargewicht von Glycerin 92 g/mol beträgt.

10. Adhäsionsmaterial nach einem der vorhergehenden Ansprüche, bei welchem der Zuckeralkohol aus der Gruppe der Alkohole, bestehend aus Xylit oder Sorbit, ausgewählt ist und der Zucker aus der Gruppe der Kohlenhydrate, bestehend aus Saccharose, Lactose, Maltose oder Fructose, ausgewählt ist.

11. Adhäsionsmaterial nach einem der vorhergehenden Ansprüche, bei welchem das Molekulargewicht der Laurinsäure/Oleinsäure zwischen 200 und 300 g/mol liegt.

12. Adhäsionsmaterial nach Anspruch 3, ferner umfassend 8 bis 12 Gew.-% von, mit Maleinsäureanhydrid gepfropftem Polypropylen oder mit Maleinsäureanhydrid gepfropftem Polyethylen oder Poly(ethylen-alt-maleinsäureanhydrid) oder Polystyrol-Block-Poly(ethylen-ran-butylen)-Block-Polystyrol-Pfropf-Maleinsäureanhydrid oder Polyisopren-Pfropf-Maleinsäureanhydrid oder Poly(methylvinylether-alt-maleinsäureanhydrid) oder Poly(isobutylen-alt-maleinsäureanhydrid) oder Poly(maleicanhydrid-alt-l-octadecen) oder Poly(ethylenglykol)methylethermethacrylat Lösung.

13. Verwendung des Adhäsionsmaterials nach einem der Ansprüche 1 bis 12 in einer medizinischen Vorrichtung, wie etwa einem Adhäsionspflaster, kinesiologischen Bändern oder einem anderen Produkt, das in Berührung mit der Haut sein muss.

14. Verfahren zur Herstellung des in den Ansprüchen 1 bis 12 beschriebenen Adhäsionsmaterials, bei welchem das Polyethylenglykol, die Zitronensäure, die Ascorbinsäure, der Zuckeralkohol oder der Zucker, das Glycerin und die Laurinsäure/Ölsäure bei Temperaturen zwischen 80°C und 200°C, während 2h bis 24h, unter Verwendung von Drücken zwischen Null und 202.650 Pa (2 Atmosphären) und mit einer Mischgeschwindigkeit zwischen 1 bis 2000 U/min gemischt werden.

15. Verfahren nach Anspruch 14, bei welchem die Geschwindigkeit der Mischung zwischen Polyethylenglykol, Zitronensäure, Ascorbinsäure, Glycerin und Laurinsäure/Ölsäure mit mit Maleinsäureanhydrid gepfropftem Polypropylen oder mit Maleinsäureanhydrid gepfropftem Polyethylen oder Poly(ethylen-alt-maleinsäureanhydrid) oder Polystyrol-Block-Poly(ethylen-ran-butylen)-Block-Polystyrol-Pfropf-Maleinsäureanhydrid oder Polyisopren-Pfropf-Maleinsäureanhydrid oder Poly(methylvinylether-alt-maleinsäureanhydrid) oder Poly(isobutylen-alt-maleinsäureanhydrid) oder Poly(maleinsäureanhydrid-alt-1-octadecen) oder Poly(ethylenglykol)methylether-methacrylat-Lösung zwischen 1 U/min bis 1000 U/min bei einer Temperatur der Mischung zwischen 23°C und 100°C liegt, während 1 Minute bis 24 Stunden.

16. Adhäsionspflaster, umfassend eine Außenschicht aus einem wasserdichten und atmungsaktiven Material, eine Zwischenschicht aus einem gewebten Stoff und eine Innenschicht aus dem Adhäsionsmaterial nach einem der Ansprüche 1 bis 12.

17. Adhäsionspflaster nach Anspruch 16, bei welchem die Außenschicht mindestens eines von ePTFe, Silikon, TPU, PEBA oder EVA ist und/oder die Zwischenschicht aus einem von einem nicht-gewebten oder gewebten Stoff besteht.

## Revendications

1. Matériau d'adhésion pour lier un objet à la peau d'un humain ou d'un animal, le matériau d'adhésion comprenant un mélange de polyéthylène glycol, acide citrique, acide ascorbique, glycérol, au moins un sucre ou un alcool de sucre, ainsi qu'un acide laurique ou un acide oléique.

2. Matériau d'adhésion selon la revendication précédente, comprenant en outre de la lignine.

3. Matériau d'adhésion selon la revendication 1 ou 2, comprenant en outre du polypropylène greffé à de l'anhydride maléique ou du polyéthylène greffé à de l'anhydride maléique ou du poly(éthylène-alt-anhydride maléique) ou polystyrène-bloc-poly(éthylène-ran-butylène)-bloc-polystyrène-greffé- anhydride maléique ou polyisoprène-greffé-anhydride maléique ou poly(éther méthylvinylique-alt-anhydride maléique) ou poly(isobutylène-alt-anhydride maléique) ou poly(anhydride maléique-alt-1-octadécène) ou solution de méthacrylate de po-ly(éthylèneglycol)-éther méthylique.

4. Matériau d'adhésion selon l'une quelconque des revendications précédentes, dans lequel 44% à 56% en poids est le mélange de polyéthylène glycol, acide citrique, acide ascorbique, glycérol xylitol et acide laurique/acide oléique ou le matériau résultant de la réaction entre eux et 44% à 56% en poids est du polypropylène greffé à de l'anhydride maléique ou du polyéthylène greffé à de l'anhydride maléique ou poly(éthylène-alt-anhydride maléique) ou polystyrène-bloc-poly(éthylène-ran-butylène)-bloc-polystyrène-greffé-anhydride maléique ou polyisoprène-greffé-anhydride maléique ou poly(éther méthylvinylique-alt-anhydride maléique) ou poly(isobutylène-alt-anhydride maléique) ou poly(anhydride maléique-alt-1-octadécène) ou solution de méthacrylate de poly(éthylène glycol)-éther méthylique.

5. Matériau d'adhésion selon l'une quelconque des revendications précédentes comprenant en outre :
- 14% à 16% en poids de polyéthylène glycol 400 ;
- 18% à 21% en poids d'acide citrique ;
- 2 % à 4 % en poids d'acide ascorbique ;
- 4% à 6% en poids de glycérol ;
- 5 % à 7 % en poids de xylitol ;
- 1% à 2% en poids d'acide oléique.

6. Matériau d'adhésion selon l'une quelconque des revendications précédentes, dans lequel le poids moléculaire de polyéthylène glycol est entre 200 et 8000 g/mol,

7. Matériau d'adhésion selon l'une quelconque des revendications précédentes, dans lequel le poids moléculaire de l'acide citrique est de 192 g/mol.

8. Matériau d'adhésion selon l'une quelconque des revendications précédentes, dans lequel le poids moléculaire de l'acide ascorbique est de 176 g/mol.

9. Matériau d'adhésion selon l'une quelconque des revendications précédentes, dans lequel le poids moléculaire de glycérol est de 92 g/mol.

10. Matériau d'adhésion selon l'une quelconque des revendications précédentes, dans lequel l'alcool de sucre est choisi dans le groupe des alcools constitué par le xylitol ou le sorbitol et le sucre est dans le groupe des glucides constitué par le saccharose, le lactose, le maltose ou le fructose.

11. Matériau d'adhésion selon l'une quelconque des revendications précédentes, dans lequel le poids moléculaire de l'acide laurique/acide oléique est compris entre 200 et 300 g/mol.

12. Matériau d'adhésion selon la revendication 3, comprenant en outre 8 % à 12 % en poids de polypropylène greffé à de l'anhydride maléique ou polyéthylène greffé à de l'anhydride maléique ou poly(éthylène-alt-anhydride maléique) ou polystyrène-bloc-poly(éthylène-ran-butylène)-bloc-polystyrène-greffé-anhydride maléique ou polyisoprène-greffé-anhydride maléique ou poly(éther méthylvinylique-alt-anhydride maléique) ou po-ly(isobutylène-alt-anhydride maléique) ou poly(anhydride maléique-alt-1-octadécène) ou solution de méthacrylate d'éther méthylique de poly(éthylèneglycol).

13. Utilisation du matériau d'adhésion selon l'une quelconque des revendications 1 à 12 dans un dispositif médical, tel qu'un patch d'adhésion, des bandes kinésiologiques ou tout autre produit devant être en contact avec la peau.

14. Procédé de production du matériau d'adhésion décrit dans les revendications 1 à 12, dans lequel le polyéthylène glycol, l'acide citrique, l'acide ascorbique, le sucre d'alcool ou le sucre, le glycérol et l'acide laurique/acide oléique sont mélangés à des températures comprises entre 80°C et 200°C, pendant 2h à 24h, en utilisant des pressions comprises entre zéro et 202,650 Pa (2 atmosphères) et avec une vitesse de mélange comprise entre 1 et 2000 tr/min.

15. Procédé selon la revendication 14, dans lequel la vitesse de mélange entre le polyéthylène glycol, l'acide citrique, l'acide ascorbique, glycérol et acide laurique/oléique avec du polypropylène greffé à l'anhydride maléique ou du polyéthylène greffé à l'anhydride maléique ou poly(éthylène-alt-anhydride maléique) ou polystyrène-bloc-poly(éthylène-ran-butylène)-bloc-polystyrène-greffé-anhydride maléique ou polyisoprène- greffé-anhydride maléique ou poly(éther méthylvinylique-alt-anhydride maléique) ou poly(isobutylène-alt-anhydride maléique) ou poly(anhydride maléique-alt-1-octadécène) ou solution de méthacrylate de po-ly(éthylèneglycol)-éther méthylique se situe entre 1 tr/minet 1000 tr/min , avec une température de mélange comprise entre 23°C et 100°C, pendant 1 minute à 24 heures.

16. Patch d'adhésion comprenant une couche externe faite d'un matériau imperméable et respirant, une couche intermédiaire faite d'un tissu, et une couche interne faite du matériau d'adhésion selon l'une quelconque des revendications 1 à 12.

17. Patch d'adhésion selon la revendication 16, dans lequel la couche externe est au moins l'un des éléments suivants : ePTFe, silicone, TPU, PEBA ou EVA et/ou la couche intermédiaire est faite d'un tissu non tissé ou tissé, tel que du coton.
